# EUROPEAN PATENT APPLICATION

(11) **EP 4 610 370 A1**
(43) Date of publication of application: **03.09.2025**
(21) Application number: 24382223.6
(22) Date of filing: 29.02.2024
(51) Int. Cl.: C12Q 1/6886

(54) **METHOD FOR PREDICTING, BEFORE THE INITIATION OF RADIOTHERAPY, WHETHER A PATIENT SUFFERING FROM CANCER WOULD DEVELOP RADIOTHERAPY TOXICITY OR RADIOTHERAPY SIDE EFFECTS**

(71) Applicant: Fundación Instituto de Investigación Sanitaria de Santiago de Compostela, 15706 Santiago de Compostela, A Coruña (ES); Servizo Galego De Saúde, 15703 Santiago de Compostela, A Coruna (ES); Fundación Pública Galega de Medicina Xenómica, 15706 Santiago de Compostela A Coruña (ES)
(72) Inventor: Vega Gliemmo, Ana, 15706 Santiago de Compostela (ES); López Pleguezuelos, Carlos, 15706 Santiago de Compostela (ES); Aguado Barrera, Miguel Elías, 15706 Santiago de Compostela (ES); Gómez Caamaño, Antonio, 15706 Santiago de Compostela (ES)
(74) Representative: Hoffmann Eitle

(57) **Abstract**

The present invention refers to an *in vitro* method for predicting, before the initiation of radiotherapy, whether a patient suffering from cancer would develop radiotherapy toxicity or radiotherapy side effects.

## Description

### FIELD OF THE INVENTION

The present invention refers to the medical field. Particularly, the present invention refers to an *in vitro* method for predicting, before the initiation of radiotherapy (RT), whether a patient suffering from cancer would develop RT toxicity or RT side effects.

### STATE OF THE ART

Currently, RT is one of the primary treatment protocols for managing clinically localized and locally advanced prostate cancer for instance, it is applied over 40% of prostate cancer patients with curative intent.

Radiation therapy implements ionizing radiation aiming to generate direct or indirect damage in the cancerous cells to slow down or abort tumor progression. Although advances are occurring rapidly making RT increasingly precise, radiation dose delivered in surrounding health structures of the tumor is unavoidable and can lead to life-limiting side effects.

Epidemiological data updated in 2023 revealed that 97% of RT prostate cancer patients had a 15-year survival after undergoing treatment. Considering these life expectancy rates, prostate cancer requires considering critical trade-offs between the short-term and long-term effects of treatments on urinary, bowel, and sexual function to ensure a reasonable quality of life after undergoing RT.

Therefore, there is an unmet medical need to achieve optimal patient management and precise personalization of radiotherapeutic treatment.

The present invention is focused on solving this problem through an innovative *in vitro* method, herein provided, for predicting, before the initiation of RT, whether a patient suffering from cancer would develop RT toxicity or RT side effects.

### DESCRIPTION OF THE INVENTION

### Brief description of the invention

The present invention refers to an *in vitro* method for predicting, before the initiation of RT, whether a patient suffering from cancer would develop RT toxicity or RT side effects.

The primary objective of the inventors of the present invention was to anticipate the onset of gastrointestinal or genitourinary toxicities, with the aim of safeguarding or enhancing the quality of life for cancer survivors undergoing RT. To this end, the inventors of the present invention performed the first epigenome-wide association study for the identification of prognostic peripheral blood methylation biomarkers that can differentiate long-term toxicities in prostate cancer. Particularly, the inventors of the present invention identified a deviation of the methylation rate along one or several CpGs of specific genes which can be used as reliable biomarkers in the personalization of RT treatment for enhance patient's quality of life.

Through the extensive analysis of the gene regions, five specific genes where identified (*NTM, ACAP1, IL1RL2, VOPP 1* and *AKR1E2*) with a high deviation in methylation, suggesting their potential role in the progression of RT toxicity, such as it is shown in the **Example 2** (below).

Consequently, the present invention is directed to the individual use of the methylation status of any of the genes *NTM, ACAP1, IL1RL2, VOPP1* or *AKR1E2,* or any combination thereof comprising 2, 3, 4 or 5 of the abovementioned genes, for predicting, before the initiation of RT, whether a patient suffering from cancer would develop RT toxicity or RT side effects, for precisely selecting patients suffering from cancer for RT, or for recommending whether to treat patients suffering from cancer with RT.

Precisely, **Table** 1 shows the list of five genomic regions, whose methylation status is different between cases and controls due to the presence of Differential Methylation Positions (DMPs) or Regions (DMRs).

**Table 1**

| **Genomic region GRC37 (bp)** | **Gene** | **Gene region** | **n DMPs** | **n DMRs (n CpGs)** |
|---|---|---|---|---|
| chr11:131319616-132184930 | *NTM* | Promoter + gene body | 24 | 1(3) |
| chr17:7239574-7254443 | *ACAP1* | Promoter + gene body | 14 | 1(10) |
| chr2:102803624-102844641 | *IL1RL2* | Promoter + 1^{st} exon | 7 | 1(3) |
| chr7:55595140-55641049 | *VOPP1* | Gene body | 6 | 1(3) |
| chr10:4867773-4868398 | *AKR1E2* | Promoter + 1^{st} exon | 7 | 1 (11) |

Moreover, for the sake of clarity, **Table 2** shows the chromosomal regions of **Table 1,** according to the different versions of the genome.

**Table 2**

| **Gene** | **Genomic region (bp) Assembly: GRC37** | **Genomic region (bp) Assembly: GRC38** |
|---|---|---|
| *NTM* | chr11:131319616-132184930 | chr11:131449722-132315036 |
| *ACAP1* | chr17:7239574-7254443 | chr17:7336255-7351124 |
| *IL1RL2* | chr2:102803624-102844641 | chr2:102187164-102228181 |
| *VOPP1* | chr7:55595140-55641049 | chr7:55527447-55573356 |
| *AKR1E2* | chr10:4867773-4868398 | chr10:4825581-4826206 |

**Table 3** shows Differential Methylation Regions (DMRs) and CpGs of the genes *NTM, ACAP1, IL1RL2, VOPP1* or *AKR1E2.*

**Table 3**

| **chr** | **start bp)** | **end (bp)** | **width (bp)** | **n CpG** | **Gene region** | **p-value** | **Gene** |
|---|---|---|---|---|---|---|---|
| chr11 | 131630464 | 131630648 | 185 | 3 | 1^{st} exon | 0.0025 | *NTM* |
| chrl7 | 7253189 | 7254443 | 1255 | 10 | 19^{th}, 20^{th}, 21^{st} exon-intron | 0.0063 | *ACAP1* |
| chr2 | 102803738 | 102803787 | 50 | 3 | 1^{st} exon | 0.0158 | *IL1RL2* |
| chr7 | 55516724 | 55516872 | 149 | 3 | 2^{nd} intron | 0.0232 | *VOPP1* |
| chr10 | 4867773 | 4868398 | 626 | 11 | promotor + 1^{st} exon | 0.0321 | AKR1E2 |

| | | | | | | | |
|---|---|---|---|---|---|---|---|
| *chr: chromosome; bp: base pair.* | | | | | | | |

Moreover, **Tables 4** to **Table 8** show examples of CpG sites with a deviation of the methylation rate, as compared with a pre-established reference methylation rate measured in controls subjects. So, these individual CpG sites, or combinations thereof, can be used in the context of the present invention.

**Table 4. Gene NTM**

| **CpG** | **logFC** | **P Value** |
|---|---|---|
| cg26251098 | 0.115 | 0.0467 |
| cg01057268 | 0.592 | 1.58E-04 |
| cg05210558 | 0.246 | 0.0123 |
| cg23520521 | -0.111 | 0.0408 |
| cg13906939 | -0.089 | 0.0299 |
| cg02234983 | 0.082 | 0.0474 |
| cg07777694 | -0.161 | 0.0131 |
| cg23056713 | -0.133 | 0.0199 |
| cg24093698 | 0.228 | 2.86E-04 |
| cg09294095 | -0.368 | 2.81E-06 |
| cg18009484 | -0.764 | 1.15E-07 |
| cg15277677 | -0.555 | 2.33E-05 |
| cg18377376 | 0.163 | 0.0038 |
| cg03471009 | 0.133 | 0.0445 |
| cg27481629 | 0.169 | 0.0194 |
| cg26918812 | -0.291 | 0.0036 |
| cg19717586 | -0.177 | 0.0101 |
| cg18282099 | 0.192 | 0.0321 |
| cg25002129 | -0.117 | 0.0135 |
| cg04667835 | -0.179 | 0.0138 |
| cg09364022 | -0.199 | 0.0315 |
| cg04865561 | -0.146 | 0.0132 |
| cg17660590 | 0.140 | 0.0053 |
| cg10677017 | -0.179 | 0.0142 |

Such as it can be seen in **Table 4,** along the *NTM* chromosomal region, a variation of CpG sites methylation status has been observed which is significantly associated with the phenotype. Each CpG may be hypermethylated (logFC above 0) or hypomethylated (logFC below 0) with respect to the control.

**Table 5. Gene ACAP1**

| **CpG** | **logFC** | **P Value** |
|---|---|---|
| cg14050954 | -0.214 | 0.001 |
| cg25671438 | -0.126 | 0.048 |
| cg25941228 | -0.155 | 0.004 |
| cg04253816 | -0.157 | 0.002 |
| cg08324090 | 0.218 | 0.001 |
| cg06721232 | 0.254 | 0.001 |
| cg25900902 | 0.629 | 2.03E-05 |
| cg07925670 | 0.343 | 4.31E-04 |
| cg20217592 | 0.474 | 1.09E-04 |
| cg15713546 | 0.285 | 0.002 |
| cg02448825 | 0.423 | 1.97E-04 |
| cg02676175 | 0.538 | 1.30E-04 |
| cg07148458 | 0.792 | 1.43E-04 |
| cg07520074 | 0.614 | 2.96E-05 |

**Table 6. Gene IL1RL2**

| **CpG** | **logFC** | **P Value** |
|---|---|---|
| cg18392357 | -0.144 | 0.005 |
| cg21181713 | -0.411 | 1.26E-05 |
| cg13779009 | -0.576 | 2.37E-05 |
| cg08023416 | -0.358 | 1.18E-05 |
| cg01376829 | -0.190 | 0.012 |
| cg13353330 | -0.140 | 0.030 |
| cg18132546 | 0.144 | 0.015 |

**Table 7. Gene VOPP1**

| **CpG** | **logFC** | **P Value** |
|---|---|---|
| cg03357999 | -0.433 | 3.23E-04 |
| cg25966908 | -0.412 | 5.50E-06 |
| cg03014829 | -0.670 | 1.75E-05 |
| cg24399349 | -0.171 | 0.002 |
| cg15863439 | -0.261 | 0.013 |
| cg08337633 | 0.242 | 0.007 |
| cg15470831 | 0.142 | 0.035 |
| cg15502645 | -0.115 | 0.025 |
| cg15902297 | 0.082 | 0.045 |
| cg26106590 | 0.136 | 0.048 |

**Table 8. Gene AKR1E2**

| **CpG** | **logFC** | **P Value** |
|---|---|---|
| cg09305334 | -0.139 | 7.95E-04 |
| cg19165274 | -0.457 | 6.82E-05 |
| cg20282550 | -0.500 | 1.44E-05 |
| cg19392551 | -0.453 | 6.81E-05 |
| cg13218425 | -0.570 | 2.30E-06 |
| cg00700969 | -0.436 | 9.06E-06 |
| cg12255897 | -0.462 | 4.71E-05 |

So, the first embodiment of the present invention refers to an *in vitro* method for predicting, before the initiation of RT, whether a patient suffering from cancer would develop RT toxicity or RT side effects, which comprises assessing the methylation status of the gene *NTM, ACAP1, IL1RL2, VOPP1* and/or *AKR1E2* in a biological sample obtained from the patient, wherein a deviation of the methylation rate along one or several CpGs of the gene *NTM, ACAP1, IL1RL2, VOPP1* and/or *AKR1E2,* as compared with a pre-established reference methylation rate in CpGs of the gene *NTM, ACAP1, IL1RL2, VOPP1* and/or *AKR1E2* measured in controls subjects, is an indication that the patient suffering from cancer would develop RT toxicity or RT side effects; or wherein an equivalent methylation rate of the CpGs of the gene *NTM, ACAP1, IL1RL2, VOPP1* and/or *AKR1E2,* as compared with a pre-established reference methylation rates of the CpGs of the gene *NTM, ACAP1, IL1RL2, VOPP1* and/or *AKR1E2* measured in control subjects, is an indication that the patient suffering from cancer would not develop RT toxicity or RT side effects.

The second embodiment of the present invention refers to a method for selecting patients suffering from cancer for a treatment with RT, or for recommending whether to treat patients suffering from cancer with RT, which comprises determining, before the initiation of RT, whether the patient would develop RT toxicity or RT side effects by assessing the methylation status of the gene *NTM, ACAP1, IL1RL2, VOPP1* and/or *AKR1E2* in a biological sample obtained from the patient, wherein a deviation of the methylation rate along one or several CpGs of the gene *NTM, ACAP1, IL1RL2, VOPP1* and/or *AKR1E2,* as compared with a pre-established reference methylation rates in CpGs of the gene *NTM, ACAP1, IL1RL2, VOPP1* and/or *AKR1E2* measured in controls subjects, is an indication that the patient suffering from cancer would develop RT toxicity or RT side effects and it would be recommended to reevaluate the therapeutic options or modify the RT treatment scheme/technique; or wherein an equivalent methylation rate along one or several CpGs of the gene *NTM, ACAP1, IL1RL2, VOPP1* and/or *AKR1E2,* as compared with a pre-established reference methylation rate of the CpGs of the gene *NTM, ACAP1, IL1RL2, VOPP1* and/or *AKR1E2* measured in control subjects, is an indication that the patient suffering from cancer would not develop RT toxicity or RT side effects and a treatment with RT would be recommended.

The third embodiment of the present invention refers to the *in vitro* use of the methylation status of the gene *NTM, ACAP1, IL1RL2, VOPP1* and/or *AKR1E2,* or of a kit comprising reagents for assessing the methylation status of the gen *NTM, ACAP1, IL1RL2, VOPP1* and/or *AKR1E2,* for predicting, before the initiation of RT, whether a patient suffering from cancer would develop RT toxicity or RT side effects, for selecting patients suffering from cancer for RT, or for recommending whether to treat patients suffering from cancer with RT.

The fourth embodiment of the present invention refers to RT for use in a method for treating cancer, wherein the method comprises selecting patients suffering from cancer for a treatment with RT by assessing the methylation status of the gene *NTM, ACAP1, IL1RL2, VOPP1* and/or *AKR1E2* in a biological sample obtained from the patient, wherein a deviation of the methylation rate along one or several CpGs of the gene *NTM, ACAP1, IL1RL2, VOPP1* and/or *AKR1E2,* as compared with a pre-established reference methylation rate in CpGs of the gene *NTM, ACAP1, IL1RL2, VOPP1* and/or *AKR1E2* measured in controls subjects, is an indication that the patient suffering from cancer would develop RT toxicity or RT side effects and it would be recommended to reevaluate the therapeutic options or modify the RT treatment scheme/technique; or wherein an equivalent methylation rate along one or several CpGs of the gene *NTM, ACAP1, IL1RL2, VOPP1* and/or *AKR1E2,* as compared with a pre-established reference methylation rate of the CpGs of the gene *NTM, ACAP1, IL1RL2, VOPP1* and/or *AKR1E2* measured in control subjects, is an indication that the patient suffering from cancer would not develop RT toxicity or RT side effects and a treatment with RT would be recommended.

In a preferred embodiment, the methylation status of the gene *NTM* is assessed in combination with the methylation status in at least a gene selected from: *ACAP1, IL1RL2, VOPP1* and/or *AKR1E2,* wherein a deviation of methylation rate along one or several CpGs of the genes, as compared with a pre-established reference methylation rate in CpGs of the genes measured in controls subjects, is an indication that the patient suffering from cancer would develop RT toxicity or RT side effects; or wherein an equivalent methylation rate of the CpGs, as compared with a pre-established reference methylation rate of the CpGs of the genes measured in control subjects, is an indication that the patient suffering from cancer would not develop RT toxicity or RT side effects.

In a preferred embodiment, the methylation status of the gene *NTM* is assessed in at least a CpG site located between the chromosomal positions hg19/GRCh37.p13 chr11:131319616-132184930 or hg38/GRCh38.p14 chrll:131449722-132315036, the methylation status of the gene *ACAP1* is assessed in at least a CpG site located between the chromosomal positions hg19/GRCh37.p13 chr17:7239574-7254443 or hg38/GRCh38.p14 chr17:7336255-7351124, the methylation status of the gene *IL1RL2* is assessed in at least a CpG site located between the chromosomal positions hgl9/GRCh37.pl3 chr2:102803624-102844641 or hg38/GRCh38.pl4 chr2:102187164-102228181, the methylation status of the gene *VOPP1* is assessed in at least a CpG site located between the chromosomal positions hg19/GRCh37.p13 chr7:55595140-55641049 or hg38/GRCh38.p14 chr7:55527447-55573356, and/or the methylation status of the gene *AKR1E2* is assessed in at least a CpG site located between the chromosomal positions hg19/GRCh37.p13 chr10:4867773-4868398 or hg38/GRCh38.p14 chr10:4825581-4826206 (see **Table 1** and **Table 2**).

In a preferred embodiment, the methylation status of the gene *NTM* is assessed in at least a CpG site of **Table 4** or any combination thereof, the methylation status of the gene *ACAP1* is assessed in at least a CpG site of **Table 5** or any combination thereof, the methylation status of the gene *IL1RL2* is assessed in at least a CpG site of **Table 6** or any combination thereof, the methylation status of the gene *VOPP1* is assessed in at least a CpG site of **Table 7** or any combination thereof, and/or the methylation status of the gene *AKR1E2* is assessed in at least a CpG site of **Table 8** or any combination thereof.

In a preferred embodiment, the biological sample is selected from: urine, saliva, plasma, blood, or serum.

The present invention may be used in any patient suffering from cancer because alterations in the *NTM* gene can affect smooth muscle repair, which could explain RT toxicity in different organs. In a preferred embodiment, the patient suffering from cancer is a patient suffering from prostate cancer.

Alternatively, the present invention refers to:
A method for identifying biomarker signatures for predicting, before the initiation of RT, whether a patient suffering from cancer would develop RT toxicity or RT side effects, for selecting patients suffering from cancer for RT, or for recommending whether to treat patients suffering from cancer with RT, which comprises assessing the methylation status of the genes, genomic regions or CpG sites disclosed in **Table 1** to **8,** wherein the identification of a statically significant deviation of the methylation status is indicative that the methylation status of the genes, genomic regions or CpG sites of **Table 1** to **8** may be used as biomarker signature for predicting, before the initiation of RT, whether a patient suffering from cancer would develop RT toxicity or RT side effects, for selecting patients suffering from cancer for RT, or for recommending whether to treat patients suffering from cancer with RT.

In a preferred embodiment, the present invention is a computer-implemented invention, wherein a processing unit (hardware) and a software are configured to: a) Receive the methylation status of the genes, genomic regions or CpG sites disclosed in **Table 1** to **8,** b) process the methylation status for finding substantial variations or deviations, and c) provide an output through a terminal display of the variation or deviation of the methylation status, wherein the variation or deviation of the methylation status may be used for predicting, before the initiation of RT, whether a patient suffering from cancer would develop RT toxicity or RT side effects, for selecting patients suffering from cancer for RT, or for recommending whether to treat patients suffering from cancer with RT.

For the purpose of the present invention, the following terms are defined:
- "CpG site": The CpG sites refers to dinucleotides formed by a cytosine followed by a guanine. Cytosines in CpG dinucleotide can be methylated to form 5-methylcytosines.
- "CpG island" (CGI): CpG islands (or CG islands) are regions with a high frequency of CpG sites. The usual formal definition is a region with at least 200 bp and a GC percentage greater than 50%.
- "Bisulfite conversion": Treatment of DNA with sodium bisulfite that converts unmethylated cytosine to uracil, which is subsequently converted to thymine during PCR amplification, while methylcytosine remains unchanged, as cytosine.
- "Promoter region": The promoter of a gene is a genomic region that extends from 1,500 bp upstream the gene to its Transcription Starting Site.
- As used herein, the term "methylation" will be understood to mean the presence of a methyl group added by the action of a DNA methyl transferase enzyme to a cytosine base followed by a guanine (CpG) nucleotide of a nucleic acid.
- Accordingly, the term, "methylation status" as used herein refers to the presence or absence of methylation in a specific nucleic acid region.
- The expression "deviation of the methylation rate" refers to a statistically significant higher/lower level of methylation (i.e., a statistically significant increase/decrease in the relative amount of methylation of a nucleic acid), as compared with a pre-established level measured in control subjects.
- The expression "equivalent methylation rate" refers to a level of methylation similar to the level measured in control subjects. In other words, this is a situation wherein there is not a statistically significant deviation of the level of methylation.
- The expression "control subjects" refers to patients suffering from cancer who underwent RT and had no toxicity within 5 years of completing their treatment compared to their initial symptoms prior to RT.
- The term "comprising" means "including", but not limited to what follows the term "comprising". Thus, the use of the term "comprising" indicates that the elements listed are necessary or mandatory, but that other elements are optional and may or may not be present.
- The term "consisting of" means "including" but is limited to what follows the term "consisting of'. Thus, the term "consists of" indicates that the elements listed are mandatory, and that other elements may not be present.
- The term "RT toxicity or RT side effects" pertains to the common general knowledge. RT employs ionizing radiation to target tumoral tissue and abort tumour progression. Nevertheless, normal tissue exposed to RT may also be affected, resulting in toxic effects. The adverse effects of RT can manifest as short-term toxicity and long-term consequences, the severity of which is highly dependent on the specific tissue being targeted. Some of the most frequent adverse effects include: Gastrointestinal damage, genitourinary damage, tissue fibrosis, vascular damage, neural damage, tissue atrophy and necrosis, cardiac toxicity, cognitive impairment, reproductive disorders, deformity and impairments to bone growth, hair loss, secondary malignancy and others [De Ruysscher, D., Niedermann, G., Burnet, N. G. et al. Radiotherapy toxicity. Nat Rev Dis Primers 5, 13 (2019). https://doi.org/10.1038/s41572-019-0064-5]*.*

### Description of the figures

**Figure 1****.** Cell type proportion comparison between cases (red) and controls (light blue). The x-axis represents cell types, and the y-axis count proportions. B: lymphocytes B; CD4T: CD4+T lymphocytes; CD8T: CD8+T lymphocytes; Mono: monocytes; Neutro: neutrophils; NK: natural killer cells.
**Figure 2****.** Volcano plot of the 681806 CpG evaluated. The x-axis represents the log10 expression fold change between cases and controls and the y-axis the log10 adjusted significance p-value.
**Figure 3****.** Heatmap including unsupervised clustering of the significant 56 DMPs. In a color gradient representing methylation rates, blue represents low methylation while red high methylation.

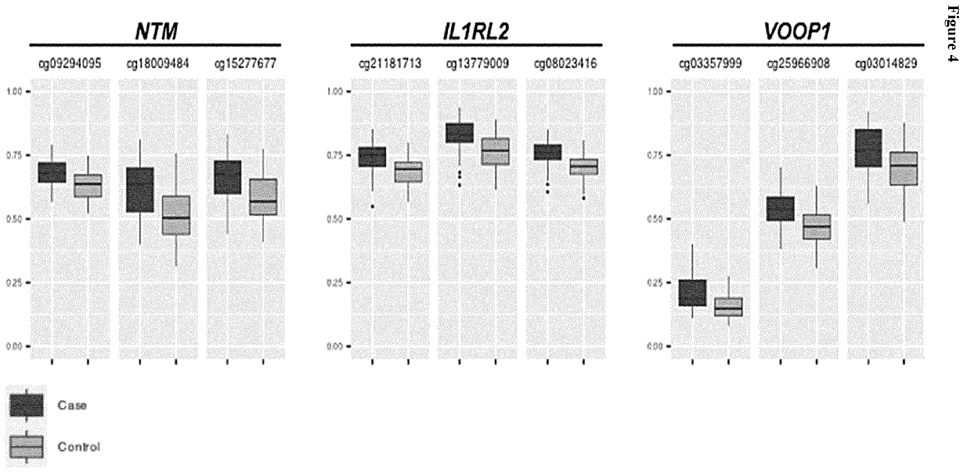

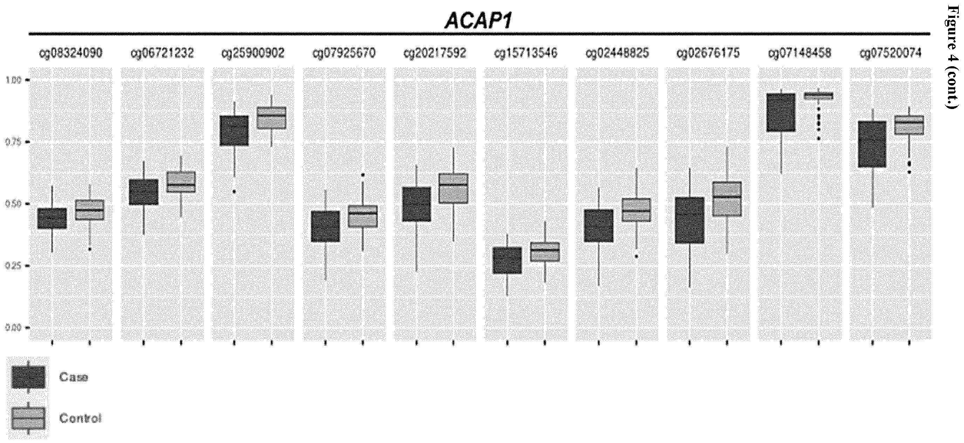

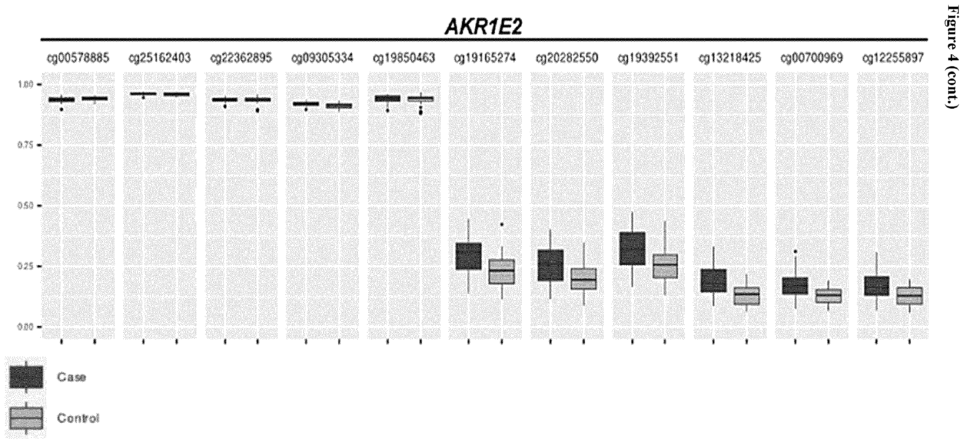

**Figure 4****.** Boxplots of the CpGs that conforms 5 DMRs. The y-axis represents the methylation rates.

### Detailed description of the invention

The present invention is illustrated by means of the Examples set below without the intention of limiting its scope of protection.

### Example 1. MATERIAL AND METHODS

### Example 1.1. Study subject and design

Samples for this study were obtained from prospective selected Galician prostate cancer patients who were treated at the Radiation Oncology Department of the Clinical University Hospital of Santiago de Compostela (northwest Spain) between 2014 and 2018. Patients treated with radical, salvage or with adjuvant RT received a total dose for the planning target volume (PTV) in the ranges of 70-76Gy, 66-70 Gy and 60-66 Gy, respectively. All individuals were recruited into the RADIOGEN-PrCa study, initiated in 2005 as a collection of blood samples, dosimetry data as well as clinical information and toxicity data. Written informed consent was obtained for each subject, according to the protocols approved by the ethics review board of the Galician Ethical Committee for Clinical Research.

Toxicity evaluation was conducted before the initiation of treatment, 6- and 12-months postcompletion of RT, and annually thereafter, following the Common Terminology Criteria for Adverse Events v4.0 (CTCAE). In this case-control study of 105 individuals, 53 served as controls, and 52 as cases. Cases were defined as patients who displayed a maximum CTCAE toxicity score adjusted for baseline ≥ 2 from month 12 to 72 after finishing RT treatment. In contrast, controls were individuals who reported a baseline-adjusted CTCAE toxicity score = 0 throughout the entire 5-year follow-up period. The following gastrointestinal and genitourinary toxicities were assessed: proctitis, bowel perforation, bowel obstruction, bowel fistula, bowel stenosis, bowel ulceration, diarrhoea, flatus, rectal bleeding, management of sphincter control, haematuria, urinary tract obstruction, urinary incontinence, urinary frequency, urinary urgency, urinary retention, urinary fistula, and urinary obstruction.

### Example 1.2. DNA isolation and methylation profiling

Blood samples were collected in 10 ml EDTA tubes until processing. DNA was isolated using magnetic bead technology with the Chemagic^{™} Magnetic Separation Module (MSM) I robot (Chemagen Biopolymer-Technologie AG). The Chemagic DNA Blood 200 Kit (PerkinElmer Inc.) was employed following the protocol specified by the manufacturer.

Bisulfite conversion was performed using the EZ DNA Methylation-Gold Kit (Zymo Research). Processed samples were hybridized to the Illumina Infinium MethylationEPIC BeadChip and scanned using the Illumina iScan platform. The intensity of the images was extracted with the GenomeStudio Methylation Software Module (v1.9.0). The samples' location in the Illumina Infinium Methylation EPIC array was randomized by age and phenotype to avoid batch effects.

### Example 1.3. Statistical analysis

Methylation data underwent quality control, normalization, and further statistical analyses using the statistical R software and Bioconductor packages. We utilized the ChAMP (Chip Analysis Methylation Pipeline) method. The raw intensity files (IDAT) were imported into the R environment using the Bioconductor package ChAMP, and subsequently transformed into β and M-values matrices. M-values were employed in our statistical analysis to circumvent issues associated withβs-values when applying linear regression. These M-values were calculated as the log2 ratio of intensities between methylated and unmethylated probes per CpG, yielding values within the range of -1 to 1.

M-values were employed in our statistical analysis to circumvent issues associated with β-values when applying linear regression. These M-values were computed as the log2 ratio of intensities between methylated and unmethylated probes for each CpG, yielding values within the range of -1 to 1.

### Example 1.4. Quality control and normalization

Patients with CpG island detection below 96% were excluded from the study. Methylation data underwent filtering to remove probes that could introduce noise and uncertainty. Low-quality probes (< 3 beads in > 5% of samples) or with detection p-values > 0.01 were filtered out. Additionally, non-CpG, non-autosomal, multi-hitting, or containing Single Nucleotide Polymorphisms (SNPs) probes were also removed.

The Illumina Infinium MethylationEPIC BeadChip employs two types of hybridization chemistries: Type I and Type II. We implemented the beta-mixture quantile normalization (BMIQ) method in ChAMP to normalize type I and II probe density distributions and avoid biased detection of Differentially Methylated Probes (DMPs).

### Example 1.5. Batch effect identification and correction

The batch effect was identified using the Singular Value Decomposition (SVD) method. The methylation matrix underwent correction for the sequencing round, sample frozen state, and sequencing array using the ComBat method to mitigate the batch effect. Additionally, a correction approach was implemented (ChAMP) to prevent the introduction of false methylation signals. This verification ensured that technical and biological variations were not confounded, thereby minimizing the occurrence of false signals.

### Example 1.6. Differential methylation analyses

To identify the Differentially Methylated Positions (DMPs) between cases and controls, we utilized the limma package, assuming a linear regression model with M-values as the dependent variable and, age and cell type proportions were included as covariates. We applied an empirical eBayes method to moderate standard errors. DMPs were then filtered using a significance threshold of adjusted P-value < 0.05 (Benjamini-Hochberg method). The results were further annotated using the DMRcate package. For the identification of Differentially Methylated Regions (DMRs), which are defined as regions with a minimum length of 1000 bp containing at least two CpGs and an F statistic <= 0.05, we employed the DMRcate package.

To perform gene set enrichment analysis (GSEA) on DMPs, we utilized the empirical Bayes GSEA method implemented in ChAMP. Additionally, we employed the R package methylGSA to extract their corresponding enriched biological functions and pathways from Gene Ontology, and Reactome databases. For all three analyses, p values were adjusted through a Benjamini-Hochberg procedure. We considered a significant enrichment if false discovery rate (FDR) < 0.05.

### Example 2. RESULTS

### Example 2.1. Differential Methylation Analyses

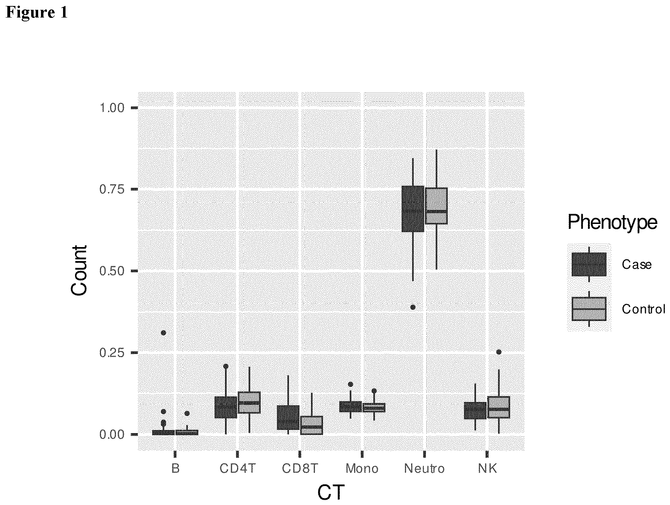

The raw methylation data underwent quality control (QC), wherein patients with a GpC island detection below 96% were excluded from the analysis. A total of 681,806 out of 850,000 probes successfully passed the QC filters. Per-sample cell-type proportions (CTp) were estimated, and no significant differences were observed for any cell type except CD8T (p-value = 0.0114) when applying a t-test between cases and controls (**Figure 1**).

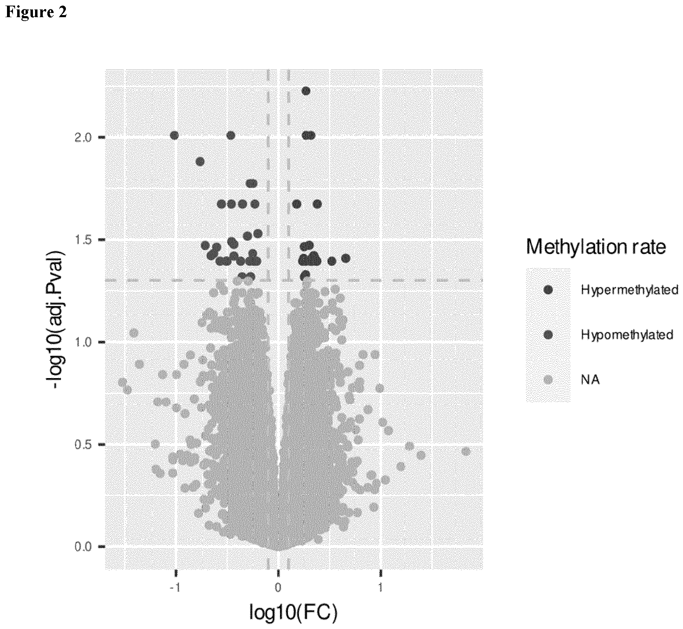

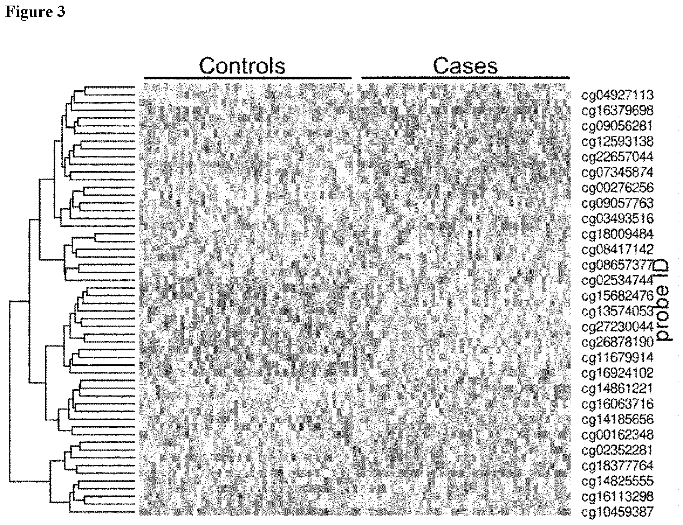

After adjusting our linear model for all CTp deconvoluted and patients' age, we identified 56 differentially methylated positions (DMPs) associated with 43 unique genes (**Figure 2**). Among these, 15 DMPs did not reside within any gene region, and 3 DMPs (cg00162348: chrl6, 30773695pb | cg11679914: chr7, 99662323pb | cg12593138: chr12, 53439997pb) were situated at positions overlapping two genes. 40.60% of the DMPs were located within the upstream region of genes (n = 8 at -1500pb and n = 20 at -200pd), 17.4% (n = 12) in the 5'-UTR, 4.3% (n = 3) in the 1^{st} exon, 31.9% (n = 22) in the gene body region, and the remaining 5.8% (n = 4) in the 3'-UTR. Out of these 56 DMPs, 31 exhibited higher methylation levels in cases, while the other 25 were higher in controls (**Figure 3**).

We identified 5 differentially methylated regions (DMRs) with a Fisher's χ² statistic p-value below 0.05 after the combined probability test, where adjacent CpG probes exhibited varying methylation levels between cases and controls. The DMR linked to the gene neurotrimin (*NTM*) (p-value = 0.0025) encompassed a 185 bp-wide region within the first intron, containing 3 hypermethylated CpG probes in cases. The second most significant DMR, with a p-value of 0.0063, displayed hypomethylation in cases across all 10 CpG probes within a genomic region (width = 1255 bp) covering exons and introns 19, 20, and 21 of the ArfGAP with coiled-coil domain-containing gene (*ACAP1*)*.* Another DMR, characterized by a p-value of 0.0158 and a length of 50 bp, exhibited hypermethylation in cases across 3 CpGs within the first intron of interleukin 1 receptor-like 2 gene (*IL1RL2*)*.* Additionally, a 149 bp-long DMR with a p-value of 0.0232, containing 3 CpGs, was identified as hypermethylated in cases within the second intron of WW domain-binding protein (*VOOP1*) gene. The final DMR within a gene region (p-value = 0.0321, width = 626 bp, and n CpGs = 11) was found, wherein all but two CpG probes displayed hypermethylation in controls within the upstream region and first exon of aldo-keto reductase family 1 member E2 gene (*AKR1E2*)*.*

## Claims

1. *In vitro* method for predicting, before the initiation of radiotherapy, whether a patient suffering from cancer would develop radiotherapy toxicity or radiotherapy side effects, which comprises assessing the methylation status of the gene *NTM* in a biological sample obtained from the patient, wherein a deviation of the methylation rate along one or several CpGs of the gene *NTM,* as compared with a pre-established reference methylation rate in CpGs of the gene *NTM* measured in controls subjects, is an indication that the patient suffering from cancer would develop radiotherapy toxicity or radiotherapy side effects; or wherein an equivalent methylation rate of the CpGs of the gene *NTM,* as compared with a pre-established reference methylation rates of the CpGs of the gene *NTM* measured in control subjects, is an indication that the patient suffering from cancer would not develop radiotherapy toxicity or radiotherapy side effects.

2. *In vitro* method for selecting patients suffering from cancer for a treatment with radiotherapy, or for recommending whether to treat patients suffering from cancer with radiotherapy, which comprises determining, before the initiation of radiotherapy, whether the patient would develop radiotherapy toxicity or radiotherapy side effects by assessing the methylation status of the gene *NTM* in a biological sample obtained from the patient, wherein a deviation of the methylation rate along one or several CpGs of the gene *NTM,* as compared with a pre-established reference methylation rates in CpGs of the gene *NTM* measured in controls subjects, is an indication that the patient suffering from cancer would develop radiotherapy toxicity or radiotherapy side effects and it would be recommended to reevaluate the therapeutic options or modify the radiotherapy treatment scheme/technique; or wherein an equivalent methylation rate of the CpGs of the gene *NTM,* as compared with a pre-established reference methylation rate of the CpGs of the gene *NTM* measured in control subjects, is an indication that the patient suffering from cancer would not develop radiotherapy toxicity or radiotherapy side effects and a treatment with radiotherapy would be recommended.

3. *In vitro* use of the methylation status of the gene *NTM,* or of a kit comprising reagents for assessing the methylation status of the gen *NTM,* for predicting, before the initiation of radiotherapy, whether a patient suffering from cancer would develop radiotherapy toxicity or radiotherapy side effects, for selecting patients suffering from cancer for radiotherapy, or for recommending whether to treat patients suffering from cancer with radiotherapy.

4. *In vitro* method or *in vitro* use, according to any of the previous claims, which comprises determining the methylation status of the gene *NTM* in combination with the methylation status at least a gene selected from: *ACAP1, IL1RL2, VOPP1* and/or *AKR1E2,* wherein a deviation of methylation rate along one or several CpGs of the genes, as compared with a pre-established reference methylation rate in CpGs of the genes measured in controls subjects, is an indication that the patient suffering from cancer would develop radiotherapy toxicity or radiotherapy side effects; or wherein an equivalent methylation rate of the CpGs of the genes, as compared with a pre-established reference methylation rate of the CpGs of the genes measured in control subjects, is an indication that the patient suffering from cancer would not develop radiotherapy toxicity or radiotherapy side effects.

5. *In vitro* method or *in vitro* use, according to any of the previous claims, wherein the methylation status of the gene *NTM* is assessed in at least a CpG site located between the chromosomal positions hg19/GRCh37.p13 chr11:131319616-132184930 or hg38/GRCh38.p14 chr11:131449722-132315036, the methylation status of the gene *ACAP1* is assessed in at least a CpG site located between the chromosomal positions hg19/GRCh37.p13 chr17:7239574-7254443 or hg38/GRCh38.p14 chr17:7336255-7351124, the methylation status of the gene *IL1RL2* is assessed in at least a CpG site located between the chromosomal positions hg19/GRCh37.p13 chr2:102803624-102844641 or hg38/GRCh38.p14 chr2:102187164-102228181, the methylation status of the gene *VOPP1* is assessed in at least a CpG site located between the chromosomal positions hg19/GRCh37.p13 chr7:55595140-55641049 or hg38/GRCh38.p14 chr7:55527447-55573356, and/or the methylation status of the gene *AKR1E2* is assessed in at least a CpG site located between the chromosomal positions hg19/GRCh37.p13 chr10:4867773-4868398 or hg38/GRCh38.p14 chr10:4825581-4826206 (see **Table 1** and **Table 2**).

6. *In vitro* method or *in vitro* use, according to any of the previous claims, wherein the methylation status of the gene NTMis assessed in at least a CpG site of **Table 4,** the methylation status of the gene *ACAP1* is assessed in at least a CpG site of **Table 5,** the methylation status of the gene *IL1RL2* is assessed in at least a CpG site of **Table 6,** the methylation status of the gene *VOPP1* is assessed in at least a CpG site of **Table 7,** and/or the methylation status of the gene *AKR1E2* is assessed in at least a CpG site of **Table 8.**

7. *In vitro* method or *in vitro* use, according to any of the previous claims, wherein the biological sample is selected from: urine, saliva, plasma, blood, or serum.

8. *In vitro* method or *in vitro* use, according to any of the previous claims, wherein the patient suffering from cancer is a patient suffering from prostate cancer.

9. Radiotherapy for use in a method for treating cancer, wherein the method comprises selecting patients suffering from cancer for a treatment with radiotherapy by assessing the methylation status of the gene *NTM* in a biological sample obtained from the patient, wherein a deviation of the methylation rate along one or several CpGs of the gene *NTM,* as compared with a pre-established reference methylation rate in CpGs of the gene *NTM* measured in controls subjects, is an indication that the patient suffering from cancer would develop radiotherapy toxicity or radiotherapy side effects and it would be recommended to reevaluate the therapeutic options or modify the radiotherapy treatment scheme/technique; or wherein an equivalent methylation rate of the CpGs of the gene *NTM,* as compared with a pre-established reference methylation rate of the CpGs of the gene *NTM* measured in control subjects, is an indication that the patient suffering from cancer would not develop radiotherapy toxicity or radiotherapy side effects and a treatment with radiotherapy would be recommended.

10. Radiotherapy for use, according to claim 9, in a method for treating prostate cancer.

11. Radiotherapy for use, according to any of the claims 9 or 10, which comprises determining the methylation status of the gene *NTM* in combination with the methylation status at least a gene selected from: *ACAP1, IL1RL2, VOPP1* and/or *AKR1E2,* wherein a deviation of methylation rate along one or several CpGs of the genes, as compared with a pre-established reference methylation rate in CpGs of the genes measured in controls subjects, is an indication that the patient suffering from cancer would develop radiotherapy toxicity or radiotherapy side effects; or wherein an equivalent methylation rate of the CpGs of the genes, as compared with a pre-established reference methylation rates of the CpGs of the genes measured in control subjects, is an indication that the patient suffering from cancer would not develop radiotherapy toxicity or radiotherapy side effects.

12. Radiotherapy for use, according to any of the claims 9 to 11, wherein the methylation status of the gene *NTM* is assessed in at least a CpG site located between the chromosomal positions hg19/GRCh37.p13 chr11:131319616-132184930 or hg38/GRCh38.p14 chr11:131449722-132315036, the methylation status of the gene *ACAP1* is assessed in at least a CpG site located between the chromosomal positions hg19/GRCh37.p13 chr17:7239574-7254443 or hg38/GRCh38.p14 chr17:7336255-7351124, the methylation status of the gene *IL1RL2* is assessed in at least a CpG site located between the chromosomal positions hg19/GRCh37.p13 chr2:102803624-102844641 or hg38/GRCh38.p14 chr2:102187164-102228181, the methylation status of the gene *VOPP1* is assessed in at least a CpG site located between the chromosomal positions hg19/GRCh37.p13 chr7:55595140-55641049 or hg38/GRCh38.p14 chr7:55527447-55573356, and/or the methylation status of the gene *AKR1E2* is assessed in at least a CpG site located between the chromosomal positions hg19/GRCh37.p13 chr10:4867773-4868398 or hg38/GRCh38.p14 chr10:4825581-4826206 (see **Table 1** and **Table 2**).

13. Radiotherapy for use, according to any of the claims 9 to 12, wherein the methylation status of the gene *NTM* is assessed in at least a CpG site of **Table 4,** the methylation status of the gene *ACAP1* is assessed in at least a CpG site of **Table 5,** the methylation status of the gene *IL1RL2* is assessed in at least a CpG site of **Table 6,** the methylation status of the gene *VOPP1* is assessed in at least a CpG site of **Table** 7, and/or the methylation status of the gene *AKR1E2* is assessed in at least a CpG site of **Table 8.**
